# EUROPEAN PATENT SPECIFICATION

(11) **EP 0 881 886 B1**
(45) Date of publication and mention of the grant of the patent: **12.03.2003**
(21) Application number: 97903716.5
(22) Date of filing: 14.02.1997
(51) Int. Cl.: A23K 1/16, A23L 1/30

(54) **ANIMAL FEED**
TIERFUTTER
ALIMENT POUR ANIMAUX

(30) Priority: 14.02.1996 SE 9600568
(43) Date of publication of application: 09.12.1998
(73) Proprietor: Biofeed (Thailand) Co., Ltd., Rayong 21150 (TH)
(72) Inventor: BUNKE, Patrik, S-753 22 Uppsala (SE); LINDBLOM, Ragnvald, 21130 Rayong (TH); KAWASHIMA, Kenji, Tatebayashi-shi, Gunma-ken 374 (JP)
(74) Representative: Kummelsten, Per-Arne
(86) International application number: SE9700252
(87) International publication number: WO97029645

(56) References cited:
- EP-A- 0 299 183
- GB-A- 1 134 206
- FILE WPI, Accession No. 96-028355, BUTTER CHEESE IND., "Prodn. of Bacterial Prepn. with Prophylactic and Medicinal Properties - for use in Animal Feeds"; & RU,C,2 035 184 (20-05-95), DW9603.

## Description

The present invention relates to a new composition containing naturally occurring substances, which are non-pathogenic living microbes, enzymes (digestive enzymes), organic acids and bacteriocins with non-toxic properties, which have multiple symbiotic effects on the digestion in animals and humans and keep the normal bacterial flora in the gut in balance. This composition is also capable of stopping existing infections and restoring a bacterial flora, which is in imbalance, to a normal condition. This has been shown by tests on a great variety of animals and humans through their own bacteriostatic and bactericide effects. Furthermore, the new composition also surprisingly acts on the feed in the stomach and in the intestine in such a way that the feed will be broken down into more easily digested fragments such as peptides and amino acids. This is most beneficial for the overall utilization of the energy-containing and other nutritional components of the feed. This composition can also surprisingly reduce the fat and the cholesterol in porc meat. This composition is also surprisingly capable of enhancing the immune defence system.

### Background of the Invention

Many different products are presently used for the treatment of animals such as pigs, piglets, sows, chickens, shrimps etc., in order to prevent bacterial infections in the stomach and the large and small intestines. The most frequently used products are various antibiotics which are usually mixed with the feed stuff and given to the animals during the whole breeding process. Also acute infections in e.g. pigs are normally treated with antibiotics.

Probiotics are sometimes used instead of antibiotics for stabilizing the intestinal micro flora and a growth promotor. The effects of the probiotics which exist today are questionable and therefore they are not regarded as a good alternative to antibiotics due to their lack of effects, or poor effects.

The effects and mode of action of the probiotics depend on the kind of micro-organism. Some of the products only consist of enzymes, e.g. the commercially available Bio-Feed Plus which is based on carbohydrate enzymes. Some of the products are based on bacilli strains and some of the products consist of various lactobacilli, streptococcus or spores from gram postive bacteria.

Common to all of them is that they are produced in such a way that they are in a purified form which means that the natural enzymes and organic acids which they produce, are washed away and the onset of the effect, when they are administered to the animals, is at either a zero level or delayed depending on a) poor or no colonization, b) the micro-organisms are destroyed in the acid part of the stomach, c) delayed production of enzymes and organic acids which means it is too late to provide growth promoting effects, d) no production of the essential lactic acids of the living microbes. Therefore, there is a definite lack of efficacy.

Pathogenic bacterial infections in the stomach and the intestine of animals, such as pigs as well as for chickens, poultry and shrimps are a great problem for the farmers as well as the consumers. Even if the farmer manages to keep a very high hygienic standard in the breeding area, it is very difficult, and if possible, to avoid such infections, and in piglets at weaning time, the development of severe diarrhea depends on immunological factors in the relationship between the natural E-coli and lactic acid in the intestine. The frequent use of antibiotics for combatting the infections has become a great problem because of the development of resistant strains of i.a. salmonella. Several of these bacteria have become resistant to many antibiotics, the result being that animals die despite the antibiotic treatment. Once the pathogenic bacteria have caused an imbalance, the animals will be unable to gain weight in a normal way due to their reduced ability to digest the feed. In addition to the obvious economic losses for the breeders, the quality of the meat is often reduced and there is an obvious risk that consumers of the meat may become infected.

One group of animals which are very sensitive to bacterial infections are piglets which quite often become infected from the sow. When they are removed from the sow, at the weaning time, and start eating solid feed, they often develop severe intestinal problems. The problems are often so severe that some of the piglets will die. The infections also cause great economic problems for the farmers, not only because some of the piglets will die, but also because the breeding time for the infected animals becomes unduly long. The severity of the problems with sick piglets vary from country to country. In Europe about 15-30% of the animals get infected, but in tropical countries the figures are considerably higher, e.g. up to 40%.

Breeding of broilers is another group with huge problems. Infections of salmonella and campylobacter in the birds are very frequent, cause the farmers big economical losses and can cause severe to lethal infections in the consumers of the chickens. The salmonella infections in broilers and laying hens are progressing into an uncontrollable stage over the whole world as the salmonella bacteria develop a high degree of resistance to all kind of antibiotics.

Once an animal has been infected by pathogenic bacteria it is difficult, or even impossible, to cure the animal with the antibiotics and probiotics which are available on the market today.

Shrimp farming is also experiencing huge problems, depending on bacterially infected water, a deteriorated bottom layer in the ponds caused by uneaten feed, excrements and chemicals. All these environmental and pollution factors increase the risk of pathogenic infections in the shrimps which causes huge economic drawbacks for the farmers. One of the most common pathogenic bacteriae which cause up to 100% mortality are different varieties of vibrio bacteriae.

From the environmental and pollution point of view the farming of chickens, laying hens and pigs present huge problems so it is urgent to reduce the amount of ammonia emissions. Emission of ammonia from the faeces on the floor is very toxic for the animals and increases the risk for infection, increases the mortality and decreases the growth performance.

Theoretically, the probably best way of solving the above mentioned problems with infections, growth promotion without antibiotics, and improved ecology would be to find a way of preventing the pathogenic bacteria from adhering to the mucous membrane surfaces, or for animals already suffering from imbalance in the bacterial flora, to find a way of restoring a normal bacterial flora which results in a decreased mortality rate of the animals and increased growth rate by using a composition of a natural "cocktail" having multiple symbiotic effects, consisting of living micro-organisms, its inherent digestive enzymes, its inherent organic acids and its inherent bacteriocins, with a quick onset and thereby strengthening of the immune defence system. Furthermore, the product should consist of the enzymes, organic acids and bacteriocins which are produced by the living micro-organism during the fermentation process. The quick onset of the multiple symbiotic actions of the enzymes, organic acids and bacteriocins, which are metabolic substances from living micro-organisms, is needed for a probiotic to be considered as effective.

### Summary of the Invention

In a first aspect the present invention relates to an animal feed additive, or composition, which is characterized in that it comprises at least three, and preferably seven, of following living micro-organisms:
a) Pediococcus pentosaceus
b) Pediococcus acidilactici
c) Picia farinosa
d) Dekkera bruxellensis
e) Bacilli
f) Streptococci
g) Staphylococci

The composition according to the invention is characterized in that it comprises enzymes "digestive enzymes" exhibiting a variety of hydrolytic activies such as the following:
h) Proteolytic enzymes, e.g. trypsin- and peptidase-like activites
i) Carbohydrate-splitting enzymes, e.g. amylase- and cellulase-like activites
j) Lipolytic enzymes, e.g. triacylglycerolase-like activities
k) Peroxidase enzymes, e.g. catalase-like activites
l) Transferase enzymes, e,g, acyltransferase-like activities
   and which is characterized in that it comprises at least three of following organic acids:
m) Lactic acid
n) Acetic acid
o) Succinic acid
   and which is characterized in that it comprises the following bacteriocins:
q) Pediocin A, Nisin, Pediocin AcH, P. acidilactice PAC 1.0
optionally together with animal feed which may be known per se. The mixture of micro-organisms will herein sometimes be referred to as a "stock culture". A preferred animal feed according to the invention comprises about 8 to 15% of water, about 15 to 30% of total protein, about 0.5 to 5% of total lipids, about 5 to 20% of fibres, about 8 to 20% pf ashes (minerals), about 30 to 50% of soluble non-nitrogenic matter, about 6 x 10⁴ to 3 x 10⁹ of live Pediococcus pentosaceus/gram, from about 1 x 10³ to 1 x 10⁷ of live Pediococcus acidilactici/gram, from about 2 x 10³ to 1 x 10⁸ Picia farinosa/gram, from about 2 x 10³ to 2 x 10⁸ of Dekkera bruxellensis/gram, from about 2 x 10³ to 4 x 10⁸ of Streptococcus/gram, from about 8 x 10² to 6 x 10⁷ Bacilli/gram, from about 6 x 10⁴ to 6 x 10⁷ of Staphylococci, from about 0.2% to 5% of lactic acid, from about 0.1 to 3% of acetic acid, from about 0.01% to 2% of succinic acid, and it preferably has an energy content of about 2-15 MJ/kg dry weight.

The above mentioned micro-organisms, enzymes, organic acids and bacteriocins preferably have i.a. the following properties:
1) Resistance to gastric acids
2) Resistance to heat up to a maximum of 70°C
3) Capability of being pelletized
4) Stability in fresh and salt water
5) Non-toxic for animals and humans
6) Preventing infections
7) Removing infections
8) Growth promoting
9) Good colonization
10) Bacteriocide effects
11) Immune enhancing effects
12) Decreases the ammonia in faeces and urea in the urin.

According to the invention there is provided a novel animal feed composition which has an unexpected effect both on animals having normal bacterial flora and, perhaps more importantly, on animals having a pathological bacterial flora.

The novel animal feed composition according to the invention, which will be described in more detail below, comprises as its essential components, pediococcus bacteria, bacilli, streptococci and staphylococci, yeast cells (fungi), hydrolytic or digestive enzymes, organic acids and bacteriocins.

The mechanism, by which the novel animal feed composition according to the invention acts in the animals, is not fully known, but it is at present assumed that the living micro-organisms, according to the invention, complete with the pathogenic bacteria to restore a normal balance and protect the normal bacterial flora as per the competitive exclusion concept.

The organic acids will decrease the pH in the crop, (for chickens and laying hens) and the large and small intestine and make it difficult for the pathogenic bacteria to grow. The lactic acid will also kill the pathogenic bacteria by lysis. Both the living micro-organisms and the lactic acid will by their symbiotic action stimulate the immune defence system when used in animals as well as in humans.

In the crop (for chicken), stomach and in the intestine, the digestive enzymes that already exist in the animal feed composition will act on the feed to break it down to more easily adsorbable proteins, fragments of proteins and amino acids, thus improving the overall utilization of the energy-containing and other nutritional components of the animal feed. Anyhow, the results will be that animals having bacterial problems will be able to restore a normal (gastro)intestinal balance. A further effect is that the digestive uptake from the intestinal tract will be increased, and by that reduce the dead organic material in the feed which is leading to decreased production of harmful ammonia and urea. A most important result is that the animal's immune defence system will be considerably improved and capable of efficiently combatting ongoing infections and/or infective invaders.

One effect of feeding animals with the composition according to the invention is that it restores/maintains a normal bacteria flora without causing any other problems. Another effect is that the composition is capable of preventing infections while keeping a high level of healthy bacteria in the intestine without causing other problems.

A further effect of the animal feed composition according to the invention is that it is capable of removing bacteria as well as improving the overall utilization of the energy-containing and other nutrional components of the animal feed. The above defined animal feed composition can successfully be used together with virtually any conventional animal feed and it will not be destroyed by the acid condition in the stomach.

Furthermore, the enzymes, the organic acids and bacteriocins which already exist in the composition according to the invention increase the speed of colonization of the living micro-organism in order for them to continue the production of more enzymes, organic acids and bacteriocins which are necessary for the quick replication of the living microbes.

The bacteriocins which are produced by the living micro-organism have at least two different actions: a) they are produced in order to protect the living microbes from other bacteria which can kill them, and b) by themselves they have an antibacterial effect.

The stock culture and animal feed composition according to the invention, its use and beneficial effects will be illustrated further in the following nonlimiting Examples.

### Example 1 - Preparation of Animal Feed

### Preparation of "Stock Culture"

1. 12 kg of soil which contains the above described micro-organisms are mixed with 30 kg rice bran, 1 kg of soy bean poweder and 14 litres of water. The mixture is heated to 50°C for 12 hours. After 12 hours the temperature is reduced to 35°C and the mixture is maintained at this temperature for 48 hours.
   The "stock culture" is dried to about 6% and is ready to be used for preparation of the animal feed composition.

### Preparation of the Animal Feed Composition

1. 500 kg of rice bran is mixed with 1.4 kg of the above described stock culture. Water is added to raise the moisture content to 35%.
2. The mixture is spread out in a layer of 10-15 cm and covered by plastic film in order to prevent water evaporation. The fermentation is finished after 8 days.
3. The plastic film is removed and the product is dried to about 7-9%. The product is then ready for direct use or packaging for subsequent use, e.g. in paper bags.

| **Analysis** | |
|---|---|
| Water content | 8.1% |
| Total protein | 22.7% |
| Total lipids | 3.1% |
| Fibers | 11.7% |
| Ashes (minerals) | 14.6% |
| Soluble non-nitrogenic substances | 39.8% |
| Number of live micro-organisms | 2 x 10⁵ - 3 x 10⁹ |
| Energy content | 11.4 MJ/kg dry weight |

### Examples of the Use of the Product

### 1. Piglets - Prevention of Weaning Diarrhea and Other Infections

The purpose wih the study was to investigate 1% of the composition according to the invention being mixed with normal feed stuff and start at weaning day (day 24) and to finish the trial when the piglets have an average weight of 20 kg.

| **Results:** | | |
|---|---|---|
| | **Control** | **Test Group** |
| Number of piglets | 188 | 178 |
| Average aget at starting | 24.04 days | 22.24 days |
| Average weight at starting | 6.78 kg | 6.15 kg |
| Average weight at finishing | 15.60 kg | 15.07 kg |
| Average age at finishing | 64.04 days | 58.24 days |
| Average trial period | 40.00 days | 36.00 days |
| Average growth increase | 8.82 kg | 8.92 kg |
| Average growth rate/day | 0.221 kg | 0.248 kg |
| Salmonella infected at day: | 35 | 40 |
| Number of infected piglets | 73 | 52 |
| Treatment with Norfloxazine injection at day: | 36 | No |
| Norfloxazine and Neomycin in the feed from day: | 37 | No |
| Mortality, number of piglets | 42 (58%) | 5 (10%) |

### Conclusion:

The trial could not be completely finished according to the protocol depending on the salmonella infection.

The 73 piglets in the control group which developed Salmonella infection were very sick and 42 died within a few days despite antibiotic treatments. At day 64 the veterinarian decided to sacrifice all the 188 piglets.

The piglets in the test group ongoing on the composition according to the invention had a delayed infection compared with the piglets in the control group. The severity of the invection and the diarrhea was mild and only 5 died in the test group without using any kind of injection of antibiotics as well as antibiotics mixed in the feed.

The composition also clearly showed better results to prevent the salmonella infection as well as destroy the salmonella bacteria compared with the traditional use of antibiotics.

### 2. Piglets - Prevention of Infection

The purpose with the study was to investigate the usefulness for piglets of 0.5% (5 kg of the composition/ton feed stuff) mixed with the normal feed stuff and starting at day 24 and stop the trial when the piglets reach 15 kg.

| **Results:** | | |
|---|---|---|
| | **Control** | **Test Group** |
| Number of piglets | 143 | 133 |
| Total weight | 975 kg | 954 kg |
| Average weight at weaning | 6.82 kg | 7.17 kg |
| Average age at weaning | 25.42 days | 24.08 days |
| Total weight at finishing | 2131.5 kg | 1961 kg |
| Total live piglets at finishing | 128 | 130 |
| Mortality, number of piglets | 15 (10.49%) | 3 (2.26%) |
| Average weight at finishing | 16.65 kg | 15.08 kg |
| Average age at finishing | 66.19 days | 60.78 days |
| Average trial period | 40.77 days | 36.76 days |
| Average growh rate | 9.83 kg | 7.91 kg |
| Average growth rate/day | 0.245 kg | 0.215 kg |
| ADG | 241.06 gram | 215.76 gram |
| FCR | 1.56 | 1.77 |
| FC/kg | 14.05 | 15.95 |

### Conclusion:

The control group has gained more in weight but this is basically because they were 4 days older and that the piglets growth rate is much faster during those 4 days.

The frequency of weaning diarrhea in the control group is relatively high. In the test group no diarrhea was recorded at all,

The mortality rate is substantially higher in the control group than the test group. The high mortality rate was dependent on severe E-Coli infection in the control group which occurred after the weaning period was over. The severity of E-Coli infection in the test group was very mild.

The dose of the composition of 0.5% is too low, but enough for protection against infections, but not enough to enhance the growth rate.

### 3. Sows

The purpose with the study was to investigate the usefulness of 1% of the composition according to the invention mixed with the normal feed stuff to be given to the sows 4 weeks before farrowing and up to weaning.

| **Results:** | | |
|---|---|---|
| | **Control** | **Test group** |
| Number of sows | 54 | 57 |
| Number of piglets | 554 | 567 |
| Stillborn piglets | 21 (3.79%) | 13 (2.29%) |
| Number of mummies | 5 (0.90%) | 10 (1.76%) |
| Total number of defective piglets | 7 (1.26%) | 12 (2.12%) |
| Small and defective piglets | 33 (5.96%) | 35 (6.17%) |
| Good live piglets | 521 (94.04%) | 532 (93.83%) |
| Average birth weights | 1.45 kg | 1.48 kg |
| Average piglets per litter | 9.65 | 9.33 |
| Weaning sows | 51 | 55 |
| Mortality rate at weaning | 51 (9.79%) | 26 (4.89%) |
| Weaning piglets | 470 | 506 |
| Average weight at weaning | 7.04 kg | 7.26 kg |
| Average weaning age | 27.57 days | 27.59 days |
| Weaned piglets/sow | 9.22 | 9.20 |

### Conclusion:

The average birth weight was higher for the piglets from the sows in the test group by 30 (2%) gram. The mortality rate was 9.79% in the control group to be compared with 4.89% in the test group, a difference of 51%.

The piglets in the test group had an average weight at weaning which ws 220 gram (3.1%) heavier than the control group, which is very important when they are moving into the weaning period.

The reason for the higher mortality rate in the control group is based, according to the veterinarian, on weaker piglets, and more severe diarrhea. They were also ready for reproduction between 4-7 days after day 28. When the sows are ongoing on the composition 4 weeks before as well as 4 weeks after farrowing the composition increases the immune defence system which is clearly indicated by the thicker milk as well as the protection from infection of the piglets.

### 4. Sows- Health Condition of Sows and Pre-weaning Mortality.

The purpose with the study was to investigate the usefulness of 1.5% of the composition (60 g/day/sow) mixed with the normal feed stuff compared to the health condition of the sows and the pre-weaning mortality.

| **Results:** | | |
|---|---|---|
| **Farrowing** | **Control** | **Test Group** |
| Number of sows | 10 | 10 |
| Total piglets born alive | 102 | 95 |
| Average born weight | 1.29 kg | 1.48 kg |
| Average total piglets/litter | 11.1 | 10.0 |
| Average pigs born/litter | 10.2 | 9.5 |
| Average stillborn pigs/litter | 0.5 | 0.2 |
| % Stillborn piglets | 4.5 | 2.0 |
| Average mummies/litter | 0.4 | 0.3 |
| % Mummies | 3.6 | 3.0 |
| | | |

| **Weaning** | | |
|---|---|---|
| Number of sows weaned | 10 | 10 |
| Total piglets weaned | 86.0 | 94.0 |
| Pigs weaned/litter | 8.6 | 9.4 |
| Pre-weaning mortality | 15.7% | 1.1% |
| Average weaning weight | 6.05 kg | 6.33 kg |
| Average age at weaning | 21.4 days | 21.8 days |

### Conclusion:

The average weight in the test group when farrowing is 190 gram or 15% higher than the control group, which is to be regarded as substantial.

The pre-weaning mortality of 15.7% in the control group is dependent on severe weaning diarrhea. The pre-weaning mortality in the test group of 1.1% must be judged as a direct result of the composition according to the invention which keeps the sows very healthy and that the milk contains more immuno globulins which are essential for the piglets and can protect them from infections.

The difference in average weaning weight of 280 gram or 5% is also substantial.

### 5. Pigs' Blood Analysis and General Analytical Values.

The test is performed by Japan Food Research Laboratories in cooperation with Yamaguchi University Agriculture Department.

A summary of the test is that the composition according to the invention have a beneficial effect as follows:

| Meat (red muscle/fat 85/15%) | Control animals | Test group |
|---|---|---|
| Water content | 63.4% | 64.1% |
| Protein | 18.5% | 19.5% |
| Fat | 16.8% | 15.1% |
| Cholesterol | 50.0 mg | 44.4 mg |
| Ph | 5.7 | 5.8 |

Conclusion: Breeding the slaughter pigs with the composition according to the invention the last 3 months decrease the level of cholesterol and fat substantial which must be regarded as very beneficial for the consumers. The reason for that depends on the conjugated bile acid enzymes which have ben produced during the production of the composition as well as produced from the living microbes in the composition when given to the pigs.
See Table 1.

### 6. Slaugther Pigs - Prevision of diarrhea and reduced breeding time.

The study was organized by Prozyme AB, Uppsala, Sweden, in cooperation with the Swedish University of Agriculture Sciences, Department of Animal Breeding and Genetics; Animal Health Services, Uppsala, Sweden and Farmek Slaughter-House, Uppsala, Sweden.

The test group and the control group included 27 animals each. The test group had an average initial weight of 32.6 kg and the control group had an average of 32.2 kg.

The pigs in each group were given the same amount of feed, but in the test group the animal feed contained 0.75% of the feed additive according to the invention. All of the animals were weight-checked once a week.

### Results:

### Test group, 27 animals:

Totally 5950 kg of feed stuff was used needed to reach final body weight, i.e. in an average of 220.4 kg feed stuff per pig. The first pig was slaughtered on day 62 and the last pig on day 154. In total, 2456 days were needed to reach the final body weight, i.e. an average of 94.3 days/pig. The FCR 3.03. See Table 1 below. None of the animals in this group needed medical care during the study. No side effects or abnormal behaviour were observed in the test group.

### Control group, 27 animals:

Totally 7335 kg of feed stuff was used needed to reach final body weight, i.e. in an average of 271.7 kg feed stuff per pig. The first pig was slaughtered on day 76 and the last pig on day 154. In total, 3043 days were needed to reach the final body weight, i.e. an average of 112.7 days/pig. The FCR 3.76. See Table 1 below. Seven animals in this group required medical treatments because of gastric disorders.

**Table 1**

| Data: | Test group | Control group |
|---|---|---|
| Total No of animals | 27 | 27 |
| Body weight at start | 32.6+13/-16 kg | 32.3+11/-17 kg |
| Body weight at termination | 105.2+8/-3 kg | 104.5+5/-6 kg |
| Total amount of feed stuff | 5950 kg | 7335 kg |
| Feed stuff, average per pig | 220.4 kg | 271.7 kg |
| Total No of days | 2546 days | 3043 days |
| Days, average per pig | 94.3 days | 112.7 days |
| FCR | 3.03 | 3.76 |
| Day of termination/No of pigs/final body weight | 62/3/103-105 | --- |
| | 76/6(103-110 | 76/2/103-108 |
| | 84/2/104-105 | 84/3/104-105 |
| | 91/5/103-106 | 91/2/104-105 |
| | 98/2/103-106 | 98/5/103-108 |
| | 105/4/103-109 | 105/2/103-105 |
| | 112/1/103 | 112/2/103-104 |
| | --- | 119/1/105 |
| | 126/2/102-112 | 126/3/105-109 |
| | --- | 133/1/104 |
| | 147/1/104 | 147/3/98-107 |
| | 154/1/105 | 154/3/105-106 |

### 7. Broilers - Prevention of Salmonella

The study were performed by Spelderholt DLO Institute for Animal Science and Health Agriculture Research Department DLO-NL P.P. Box 15 7360 AA Beekbergen, the Netherlands.

### Salmonella Challenge of Broilers

Broilers of 8 pens were individually infected orally at day 14 with 10⁴ cfu Salmonella infantis NaI and in the other groups 5 marked seeders per pen were placed, which simulates the natural infection. The seeders had been orally infected with 5 x 10⁷ cfu Salmonella infantis NaI at day 14. The Salmonella status of the experimental chicks before infection or placing of seeders was tested by faecal sampling. At sampling dates (1 week post infection), 5 broilers from each pen were slaughtered and in the caeca the number of Salmonella was estimated.

### Feed

In the feed 50 mg/kg Zincbacitracin as a growth promotor and 3 mg/kg halofuginone as a coccidiostat were included. Feed was prepared for the entire experimental period and was administered "ad libitum". The feed additive according to the invention was incorporated in the feed before pelleting (1% on dry weight basis). Pelleting temperature was 70°C.

### Microbiology

At 7 days post infection, from each pen 6 chicks were chosen randomly (a total of 48 chicks per group) and slaughtered. The caeca were taken out and after sterilizing the surface, a sample of the contents was taken aseptically. The cfu count of Salmonella infantis NaI was estimated by plating on "Brilliant Green Agar" (Oxoid CM 329) with 20 ppm Nalidex acid. Tenfold dilutions of caecal contents in "Buffered Peptone Water" (Oxoid CM 509) were incubated and plated on BGA agar after enrichment.

### Statistics

The results were analysed with the student t test.

### Results and Discussion

The infection of the seeders with very high doses of Salmonella (5 x 107) did establish as expected and after a few days the number of Salmonella positive checks was at an acceptable level of approximately 70%.

The mean number of Salmonella cfu in the caecal contents of broilers from both test groups was significantly lower than from its control group (P<0.01).

In Table 2 results are given from individual cfu counts in caecal contents. In the Table the number of broilers with a certain level of Salmonella cfu is indicated. From these results Infection Factors (IF) and Protection Factor (PF) can be calculated. The IF is the geometric mean of the number of Salmonella per gram of caecal contents of all chickens in a particular group. From this IF the PF can be obtained, by dividing the IF value of the control groups by that of the treated group.

**Table 2.**

| **Number of chicks with certain numbers of Salmonella cfu per gram caecal contents, IF (infection factor) and PF (protection factor)** | | | | | | | | | | | |
|---|---|---|---|---|---|---|---|---|---|---|---|
| Week 1 Orally infected. | | | | | | | | | | | |
| | number of Salmonella per gram caecal contents | | | | | | | | | | |
| | n | 0 | 10 | 10² | 10³ | 10⁴ | 10⁵ | 10⁶ | 10⁷ | >10⁸ IF | |
| control | 40 | 0 | 0 | 1 | 6 | 10 | 12 | 9 | 1 | 1 | 4.7 |
| Test | 40 | 10 | 3 | 0 | 7 | 1 | 7 | 6 | 4 | 2 | 3.6 |
| | | | | | | | | | | PF: | 1.3 |
| | | | | | | | | | | | |

| Week 2 via seeders. | | | | | | | | | | | |
|---|---|---|---|---|---|---|---|---|---|---|---|
| | number of Salmonella per gram caecal contents | | | | | | | | | | |
| | n | 0 | 10 | 10² | 10³ | 10⁴ | 10⁵ | 10⁶ | 10⁷ | >10⁸ IF | |
| control | 40 | 2 | 0 | 0 | 3 | 3 | 14 | 11 | 2 | 5 | 5.3 |
| Test | 40 | 20 | 0 | 3 | 1 | 2 | 6 | 6 | 1 | 1 | 2.5 |
| | | | | | | | | | | PF: | 2.1 |

At the sampling date (1 week p.i.), Salmonella was absent in a higher number of broilers from the test group than form the control group (Table 3).

**Table 3.**

| **Salmonella analysis of caecal samples of Salmonella negative chicks.** | | | | |
|---|---|---|---|---|
| n=40 | orally | | seeders | |
| | control | test | control | test |
| Week 1 | 0 | 10 | 1 | 20 |

### Conclusion

Administration of the composition acording to the invention to broilers significantly contributes to the early exclusion of Salmonella. The quick onset of the effect depends on the multiple symbiotic action from the living micro-organisms, the enzymes, the organic acids and bacteriocins which are the contents in the composition. The results clearly show the effectiveness of the composition for removing Salmonella infections from broilers.

### 8. Broilers - Growth promotion

The purpose with the study was to compare the composition according to the invention using 1% mixed with feed stuff which contains no antibiotics with a control group with normal feed stuff containing antibiotics for breeding of broilers one day old chickens to slaughtering. The study was performed by Chaiyaree Farm Co., Ltd. panat Nikhom, Thailand.

| **Results** | | |
|---|---|---|
| | **Control** | **Test group** |
| Number of chickens | 10 200 | 10 200 |
| Age of chickens | 1 day old | 1 day old |
| Age when finishing | 46 days | 46 days |

| Medicines used during the trials: | | |
|---|---|---|
| - Mintervit | day 1 | day 1 |
| - IB A 3 | day 2 | day 2 |
| - Tylan | day 3 | day 2 |
| - Permasol | day 6 | day 9 |
| - ND Clone | day 10 | day 8 |
| - IBD Blen | day 14 | day 13 |
| - Tylosin S.P. | day 17 | None |
| - Post-Vaccine | day 18 | day 15 |
| - Chlor-Ery | day 28 | day 28 |
| - Triane | day 40 | day 40 |

| **Dead chickens:** | | |
|---|---|---|
| At week 1 | 82 heads | 57 heads |
| At week 2 | 115 heads | 106 heads |
| At week 3 | 69 heads | 50 heads |
| At week 4 | 33 heads | 27 heads |
| At week 5 | 43 heads | 33 heads |
| At week 6 | 118 heads | 42 heads |
| At week 7 | 325 heads | 45 heads |
| **Total:** | **785 heads** | **360 heads** |

| **Sick and weak chickens, which are taken away.** | | |
|---|---|---|
| | Control | Test group |
| Week 1 | 10 heads | 26 heads |
| Week 2 | 24 heads | 20 heads |
| Week 3 | 26 heads | 6 heads |
| Week 4 | 18 heads | 0 head |
| Week 5 | 16 heads | 0 head |
| Week 6 | 26 heads | 1 head |
| Week 7 | 111 heads | 89 heads |
| **Total:** | **231 heads** | **142 heads** |

| **Total dead and sick chickens, heads:** | | |
|---|---|---|
| Week 1 | 92 (0.90%) | 83 (0.81%) |
| Week 2 | 139(1.36%) | 126(1.24%) |
| Week 3 | 95 (0.93%) | 56 (0.55%) |
| Week 4 | 51 (0.50%) | 27 (0.26%) |
| Week 5 | 59 (0.58%) | 33 (0.32%) |
| Week 6 | 144 (1.41%) | 43 (0.42%) |
| Week 7 | 436 (4.27%) | 134 (1.31%) |
| **Total:** | **1016 (9.96%)** | **502 (4.92%)** |
| Live chickens | 9184 | 9698 |
| Total kg: | 17,698.431 | 19,846.96 |
| Average weight | 1.93 kg | 2.05 kg |
| Total feed consumption kg | 36.195 | 40.489 |
| FCR | 2.05 | 2.04 |

### Conclusion:

The trial as such is very well controlled by managements from Chaiyaree Farm Co., Ltd. The chickens got the last antibiotics 6 days before slaughtering and in the control group the chickens got diarrhea and losing in weight as well as high total mortality rate.

During the last week when antibiotics are forbidden the chickens are losing weight and the mortality increases, which are a huge problems for the farmers to overcome. During the last week the risk for re-infection of Salmonella and Campylobacter occur which can spoil the whole business for the farmers.

The differences in mortality is substantial as well as the more heavy chickens in the test group.

The differences of 120 gram/chicken in combination with more live chickens are from the economical point of view very substantial.

### 9. Broiler-Cost benefit study.

### Bakcground:

SUNEK FOO LTD., Saraburi, Thailand, is a company which export broilers to Japan. They are among the top 10 companies in Thailand in this field.

Every company which exports broilers must follow very strict rules regarding residues of antibiotics, chemicals and any pathogenic bacteria in the meat.

This company has a processing production capacity of 60 000 broilers/day or about 19 millions broilers/year. This means they need to perform the broiler breeding in a very professional manner in order to maximise profits and establish a good relationship with their Japanese customers. SUNEK has a long experience testing with "probiotics" as an alternative to antibiotics for growth promoting as well as for excluding salmonella contamination of the meat. Up until now they had not found any kind of probiotics available on the market which fulfill all the criteria.

We can describe the trial conditions using the composition according to the invention as follows:

### Criteria:

10 000 one day old chickens in each group and located in 2 different houses, about 50 meters from each other.

The control group and the test group should have the same kind of antibiotic-free feed and the same standard medical treatment program. The same number of labourers should take care of the two groups. The chickens in the test group should get 0.5% (kg/1 ton feed pellets) of the composition mixed with the feed pellets. Slaughtering should be done at day 44-46 at their own slaughtering factory in Saraburi province.

| **Results:** | | |
|---|---|---|
| | **Test group** | **Control grop** |
| Number of 1 day old chickens | 10 600 | 10 000 |

| **Mortality:** | | |
|---|---|---|
| Week 1 | 119 (1.12%) | 180 (1.80%) |
| Week 2 | 131 (1.24%) | 203 (2.03%) |
| Week 3 | 112 (1.06%) | 172 (1.72%) |
| Week 4 4 | 115 (1.09%) | 138 (1.38%) |
| Week 5 | 97 (0.92%) | 211 (2.11%) |
| Week 6 | 171 (1.61%) | 186 (1.86%) |
| Week 7 | 134 (1.26%) | 78 (0.78%) |
| **Total:** | **879 (8.29%)** | **1168 (11.68%)** |
| Rem: The week 7 for the test group total 4 days and in the control group 3 days. | | |

| **Feed consumption:** | | |
|---|---|---|
| Week 1 | 1,500 kg | 1,350 kg |
| Week 2 | 3,060 kg | 2,850 kg |
| Week 3 | 5,070 kg | 4,770 kg |
| Week 4 | 7,140 kg | 6,540 kg |
| Week 5 | 7,950 kg | 7,830 kg |
| Week 6 | 9,570 kg | 9,930 kg |
| Week 7 | 4,080 kg | 2,160 kg |
| **Total:** | **38,370 kg** | **35,430 kg** |
| Rem: The week 7 for the test group total 4 days and in the control group 3 days. | | |

| **Medical program:** | | |
|---|---|---|
| | **Test group** | **Control group** |
| Day 1 | IB vaccine | IB vaccine |
| Day 1-3 | Tylosin | Tylosin |
| Day 10 | ND vaccine | ND vaccine |
| Day 14 | IBD vaccine | IBD vaccine |
| Day 15 | Amcolistine | Amcolistine |
| Day 22 | Tetramycin | Tetramycin |
| Day 29 | Tetramycin | Tetramycin |
| Day 35 | Norfloxacin | Norfloxacin |
| | | |
| Total body weight: | 18,700 kg | 16,000 kg |
| | | |
| Number of chickens to slaughtering: | 9,721 heads | 8,832 heads |
| | | |
| Total feed consumption: | 38,370 kg | 35,430 kg |
| | | |
| FCR: | 2.05 | 2.21 |
| | | |
| Average weight/chicken: | 1.92 kg | 1.81 kg |

| **Cost benefit analysis:** | | |
|---|---|---|
| **Expenses:** | **Test group** | **Control group** |
| 1 day old chickens: | 4,240 USD | 4,000 USD |
| Medicines: | 636 USD | 600 USD |
| Feed: | 9,669 USD | 8,928 USD |
| Test samples: | 191.84 USD | 0 USD |
| Labour | 212 USD | 200 USD |
| TOTAL: | 14,834.68 USD | 13,728.36 USD |
| | | |

| **Income:** | | |
|---|---|---|
| TOTAL: | 20,196 USD | 17,280 USD |
| | | |
| **Total net income:** | 5,261.32 USD | 3,551.64 USD |
| **Net income/chicken:** | 0.496 USD | 0.356 USD |

### Discussion:

The trial has been 100% performed and controlled by SUNEK FOOD LTD. Biofeed's staff have visited the farm once/week to collect the data and inspect the health condition of the chickens.

SUNEK decided to use the same standard medical program for both groups in order to get a comparative trial.

### Conclusion:

The differences between the two groups are, in all respects, significant. The mortality rate in the control group of 11.68%, compared with 8.29% in the test group is about 29% which is definitely statistically significant. The difference in FCR of 2.05 in the test group vs 2.21 in the control group is more than 7% and must also judged as substantial.

However, the most important is the cost benefit analysis which clearly shows the benefits of using the composition according to the invention and the differences could have been bigger if the use of antibiotics after day 3 had been withdrawn as that kind of antibiotic treatment is only prophylactic. However, the net profit per chicken in the test group is 12.41 Baht/chicken and in the control group is 8,89 Baht/chicken which is a difference of 40%.

The differences between the composition and other kinds of microbial products is very large depending on the fact that composition could classified as a finished "Biotic System" which from the beginning contains all the needed ingredients such as living microbes, organic acids, enzymes and bacteriocins. That together is the guarantee to get a good early colonization in the crop and a direct effect of the enzymes on the feed in the crop as well.

The organic acids will first of all decrease the pH and make the situation for pathogenic bacteria unfavourable, and that together especially with a) the lactic acid which immediately has a bacteriostatic effect and b) the overall immune enhancing effects from the living microbes is what it needed to keep the chickens in a healthy condition.

When using products which contain living microbes in chickens it is very important that the beneficial effects start directly in the crop and that there is minimal damage of the living microbes in the acid part of the stomach as the chickens have a poor digestive mechanism and a low nutritional uptake.

The composition works from the first second compared with traditional probiotics which are acting very slowly as they have to start up the production of their own metabolites before they can work.

The above mentioned mode of action is the reason for the excellent net profit level when using the composition.

We can also add that there is a decreased risk of salmonella and campylobacter infections in the chickens, when using the composition, which normally is a huge problem over the whole world.

What could also be recorded was the decreased smell of ammoniak from the test group compared with the control group.

### 10. Shrimps and Prawns

Prawns cultivated in ponds are very sensitive to bacterial infection. Once an infection occurs all of the shrimps or prawns in the ponds die. In order to minimize the risk of infection, the composition according to the invention is used in concentration of about 20 grams/Sqm which are spread out evenly over the empty bottom of the pond. The composition are acting during 7-10 days and after that time the concentration of pathogenic micro-organism are minimized to a level which do not harm the shrimps/prawns later on.

After stocking of post larvae the composition according to the invention is used twice a week with 2 kg/1600 Sqm. Additionally, 1% of fine powder (mesh 60) of the composition is also mixed with the feed pellets and given to the shrimps every day.
**Conclusion:** The shrimps in the ponds which have been treated with the composition as well as feed additive are growing to harvest size in 100 days compared with the control which averages 120 to 150 days. The yield is much higher and the protection from different infections as e.g. Vibrio bacteria, viruses like yellow head and White Spot Virus.

### 11. Shrimp-Vibrio infection.

In 2 ponds with Vibrio bacteria infection feaces samples was taken from both ponds. The amount of Vibrio bacteria in the faeces was after culture 2x10⁶ cfu/gram. In one of the pond the water was treated with the 3 kg of the composition twice a week for 2 weeks. The feed pellets was also mixed with the composition with 1 kg of composition/100 kg feed pellets. The other pond had no treatment at all.

After 2 weeks faeces was collected from the shrimps from both the ponds and cultured.

### Results:

The control pond without any treatment had 8x10⁶ cfu vibrio/gram and in the pond treated with the composition according to the invention had 2x10³ cfu vibrio/gram faeces.

### 12. Cats and Dogs

A number of both sick and healthy cats, kittens and dogs have eaten the composition according to the invention every day for more than 6 months. Animals with digestive problems have turned into a healthy condition within a few days and healthy animals keeps in good condition, resulting in normal faeces, good for condition and less smell from the animals.

### 13. Horses

50 horses to be trained as trotting horses suffering from intestinal problems resulting in loose stool, loss of weight, undernourisment, bad fur and an overall bad condition were given 60 gram/day of the feed additive according to the invention during 2 weeks. After 2 weeks the dose was increased to 180 gram/day. The feed additive was mixed with the normal feed so as to be spread out over the day.
**Results:** Already after 3 days the faeces returned to normal color and normal size. After 4 weeks the horses began to gain weight and the condition of the fur and overall condition improved. At that time the horses could start training in a normal way again.

### 14. Environmental improvement

The smells from broiler, laying hen and pig farms are huge problems as well as severe pollution problems which is a result of intensive farming. When using the composition according to the invention as a feed additive over a period more than 6 months the smells from the farms decline which easily can be detected by the reduced smell from ammonia. Already after 1 months use of the composition the condition in the farms changes drastically.

### 15. Decomposition of urine from animals

A 2 meter deep layer of sawdust was applied to a special container, 100 meters long, 6 meters wide and 5 meters deep. 50 tons of urine was sprayed over the sawdust layer and 5 kg of the composition according to the invention per 40 Sqm were added. After 5 hours the first 1 metre layer mechanically stirred up air inspired by a brower. An odorles water solution was filtered through the sawdust layer. This odorless water contains less than 100 ppm of free nitrogen and hydrogen sulphide contaminants. 50 tons of urine can be decomposed per day for 18 months. After 18 months the 1 metre upper layer of sawdust must be replaced and fresh sawdust added. This way to decompose urine is excellent in order to avoid environmental problems.

Similarly, the additive according to the invention can also be used for degrading digested sludge, and the product obtained can also be used as a fertilizer and for composting.

### 16. Human use - Acute bacterial infections

10 persons suffering from acute intestinal problems, caused by bacterial infections have been eating the composition according to the invention 3 grams 3 times/day for 1-3 days.
**Results:** 6 of the persons responded within 12 hours with no more pain and discomfort no diarrhea. 4 persons had turned to a normal situation after 24 hours.

### 17. Patients with nutritious problems

Cancer patients normally suffer from digestion problems and they cannot break down the food in a normal way which means that they get a very poor nutrition which is a big problem for the immune defence system to work. Five hospitilized patients suffering from cancer diseases resulting in heavy loss of weight and in general bad condition was given the composition according to the invention mixed with the drinking water 5 gram/day.
**Results:** After 6 days the problems with loss stool disappeared and the second week a small gain in weight could be recorded. The appetite returned and after 4 weeks the patients gain substantially in weight and in general the health condition improved.

### 18. Comparative experiments with the composition according to the invention without enzymes, organic acids and bacteriocins

The purpuse with following study was to investigate if the enzymes, the organic acids and the bacteriocins are needed in the composition according to the invention. Broilers have been used for the evaluation and using the growth rate and mortality as a method for analysis.
1. The fermented product according to the production method was washed with water and filtrated through a very fine net in order to separate the rice bran and the living micro-organism from the enzymes, the organic acids and the bacteriocins. The residue was washed with water.
No detectable level of living organisms could be detected in the water phase and no detectable level of organic acids and enzymes could be detected in the rice bran and residual living micro-organisms.
2. The fermented product according to the production method was washed with water and filtrated through a net in order to separate the rice bran from the living micro-organism the enzymes, the organic acids and the bacteriocins.
3. Following test was performed with 1 day old chickens and finished at day 45 and with 1000 chicks in each group and with the calculated same amount of micro-organism as have been used before i.e. 1% added to normal feed stuff, see Table 4:

**Table 4**

| Group | Growth rate | Mortality % |
|---|---|---|
| | average weight | |
| 1. Control (no additive according to the invention) | 1.90 kg | 10.75% |
| 2. Composition according to the invention. | 2.10 kg | 4.63% |
| 3. Composition according to the invention (without enzymes, organic acids and bacteriocins) | 1.94 kg | 8.40% |
| 4. Water phase (only enzymes, organic acids and bacteriocins) | 1.90 kg | 9.10% |
| 5. Water phase (only micro organisms, without enzymes, organic acids and bacteriocins) | 1.96 kg | 8.50% |
| 6. Water phase (containing living micro-organisms, enzymes, organic acids and bacteriocins) | 2,15 kg | 4,15% |

### Conclusion:

The trials have been performed at the same test farm and at the same time. The number of chickens (1000 heads in each group) are big enough in order to evaluate the results.

The results clearly show that the combination of living micro-organisms, enzymes, organic acids and bacteriocins according to the invention are needed for quick onset, which have resulted in increased growth rate in the test groups 2 and 6 compared with the control group 1 and the other groups. Test group 6 showed little bit better results than group 2 which can be explained that when the water solution are mixed with the normal drinking water all the chickens get the composition in a better and more easy way compared when the composition is mixed with feed pellets.

**Table 5**

| **Blood analysis of pigs after feeding with the composition for 3 months** | | | | | |
|---|---|---|---|---|---|
| Control Group | TG mg/dl | TC mg/dl | PL mg/dl | FFA µmol/l | GL mg/dl |
| 92.02.28 | 76.6 | 89.9 | 128.3 | 157 | 130.9 |
| 92.03.07 | 28.7 | 98.0 | 138.0 | 216 | 99.0 |
| 92.03.17 | 43.6 | 96.0 | 137.0 | 255 | 85.3 |
| 92.04.06 | 20.2 | 66.0 | 101.1 | 98 | 100.0 |
| 92.04.15 | 84.0 | 85.9 | 114.1 | 153 | 164.7 |
| 92.04.24 | 35.1 | 92.9 | 141.3 | 110 | 115.2 |
| 92.05.20 | 35.1 | 89.9 | 96.7 | 212 | 79.4 |

**Table 6**

| Control Group | TG mg/dl | TC mg/dl | PL mg/dl | FFA µmol/l | GL mg/dl |
|---|---|---|---|---|---|
| 92.02.28 | 53.2 | 77.8 | 107.6 | 192 | 138.7 |
| 92.03.07 | --- | --- | --- | --- | --- |
| 92.03.17 | 26.6 | 85.9 | 127.2 | 475 | 106.4 |
| 92.04.06 | 22.3 | 70.7 | 93.5 | 137 | 107.4 |
| 92.04.15 | 18.1 | 80.8 | 110.9 | 90 | 164.7 |
| 92.04.24 | 29.8 | 71.7 | 103.3 | 129 | 139.2 |
| 92.05.20 | 14.9 | 62.1 | 75.0 | 47 | 74.0 |
| TG: Triacylglycerol | | | | | |
| TC: Total Cholesterol | | | | | |
| PL: Phospholipids | | | | | |
| FFA: Free Fatty Acids | | | | | |
| GL: Glucose | | | | | |

### 19. Decay of fresh manure.

Fresh manure from pigs was divided in 5 experimental groups as:
1. Control
2. Mixed with 0.5, 1, 1.5 and 2% of the composition according to the invention.

The process was followed by measuring the pH and ammonia.

The pH was measured in the slurry 3 hours before the composition was added.

Supply of air was done with a blower every third day during the first 3 weeks.

| **pH in manure** | | | | | |
|---|---|---|---|---|---|
| Day | Control | BX-1, 0.5% | BX-1, 1.0% | BX-1, 1.5% | BX-1, 2% |
| 0 | 6.6 | 6.4 | 6.4 | 6.3 | 6.4 |
| 7 | 6.4 | 6.0 | 5.9 | 5.8 | 5.8 |
| 14 | 6.4 | 5.9 | 5.8 | 5.7 | 5.6 |
| 21 | 7.5 | 6.1 | 5.8 | 5.7 | 5.6 |

| **Ammonia (ppm) in the air about 5 cm from the manure surface** | | | | | |
|---|---|---|---|---|---|
| Day | Control | BX-1, 0.5% | BX-1, 1.0% | BX-1, 1.5% | BX-1, 2% |
| 0 | 0 | 0 | 0 | 0 | 0 |
| 14 | 55 ppm | - | - | - | 3 |
| 21 | 750 ppm | 5 ppm | 8 ppm | 4 ppm | 5 ppm |

The decay process starting within 2 hours in the test groups. After 2 weeks the smell is reduced from the test compared with the control. The ammonia was measured with Drager Gas Detector Pump accuro 21/31. The smell of manure is very strong in the control group at day zero and at day 21 intensive. The smell in the test groups at day 21 is more acid (lactic acid) and no smell of pig can be recognized.

The test clearly demonstrates the properties of the composition to reduce the production of ammonia and by that emission. It also clearly demonstrates the capacity to keep a lower pH which from the environmental point of view is extremely important. With a low pH and a low emission of ammonia the new method to use the composition with fresh manure reduces the environmental problems which are contected with manure.

### 20. Decay of old manure.

Three weeks old manure from pigs was divided in two experimental groups as:
1. Control
2. Mixed with 1% of the composition according to the invention.

The process was follwed by measuring the pH and ammonia.

The "old" manure was mixed with water to a total water concentration of about 60%.

The pH was measured in the slurry 3 hours before the composition was added. Supply of air was done with a blower every day during the first 2 weeks.

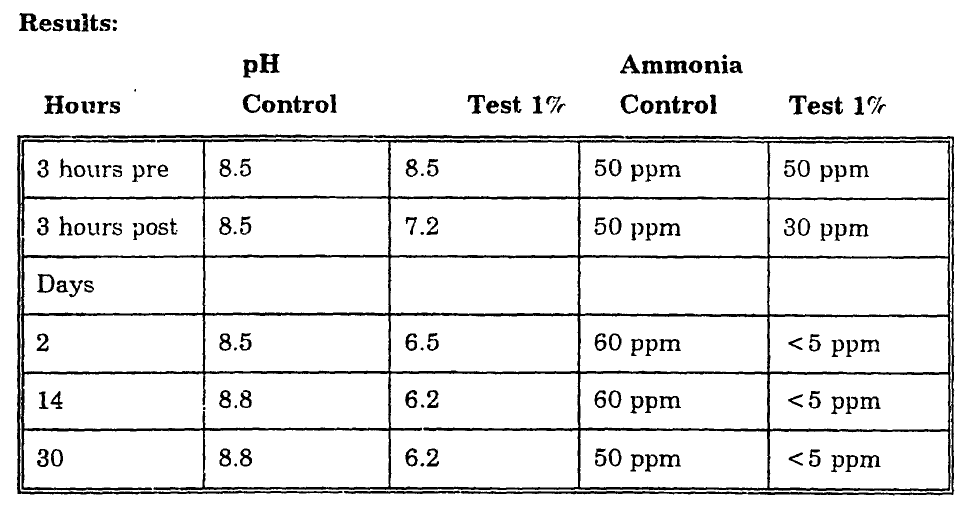

### 22. Temperature resistance of the living micro-organisms.

The purpose to heat up the composition was to investigate the metabolic activites. The composition was heated during 10 minutes to 100°C. The composition was then mixed with water and sugar and the carbon oxide production was monitored.

After 6 hours a clear fermentation of the sugar could be recognized and after 2 weeks all the sugar was fermented and production of pactic acids, acetic acid and alcohol could be detected.

The results shows that despite very high temperature the micro-organisms still have a metabolic activity to produce enzymes and organic acids.

## Claims

1. An animal feed additive for animals, **characterized in that** it comprises at least one of the following micro-organisms:
a) Pediococcus pentosaceus,
b) Pediococcus acidilactici,
and at least one of the following micro-organisms:
c) Picia farinosa,
d) Dekkera bruxellensis,
and at least one of the following micro-organisms:
e) Bacilli,
f) Streptococci,
g) Staphylococci,
and at least also one or more of the following hydrolytic enzymes:
h) Proteolytic enzymes,
i) Carbohydrate-splitting enzymes,
j) Lipolytic enzymes,
k) Peroxidase enzymes, and
l) Transferase enzymes,
and at least one of the following organic acids:
m) Lactic acid
n) Acetic acid
o) Succinic acid
and at least two of the following bacteriocins:
q) Pediocin A, Nisin, Pediocin AcH, P. acidilactice PAC 1.0

2. An animal feed additive according to claim 1 **characterized in that** it comprises all of the living micro-organisms a) - g).

3. An animal feed additive according to any one of the preceding claims, **characterized in that** it comprises all of the hydrolytic enzyms h) - l).

4. An animal feed additive according to any one of the preceding claims **characterized in that** it comprises all of the organic acids m) - o).

5. An animal feed additive according to any one of the preceding claims, **characterized in that** it comprises all of the bacteriocins Pediocin A, Nisin, Pediocin AcH and P. acidilactice PAC 1.0

6. An animal feed additive according to any one of the preceding claims, **characterized in that** said proteolytic enzymes include enzymes having trypsin- and peptidase-like activities,
said carbohydrate-splitting enzymes include amylase- and cellulase-like activities, said lipolytic enzymes include triacylglycerolase-like activities,
k) said peroxidase enzymes include catalase-like activities, and
l) said transferase enzymes include acyltransferase-like activities.

7. An animal feed additive according to any one of the preceding claims, **characterized in that** it also comprises an animal feed which may be known per se.

8. An animal feed additive according to any one of the preceding claims, **characterized in that** it comprises about 8 to 15% of water, about 15 to 30% of total protein, about 0.5 to 5% of total lipids, about 5 to 20% of fibres, about 8 to 20% of ashes (minerals), about 30 to 50% of soluable non-nitrogenic matter, about 6 x 10⁴ to 3 x 10⁹ live Pediococcus pentosaceus per gram, from about 1 x 10³ to 1 x 10⁷ live Pediococcus acidilactici per gram, from about 2 x 10³ to 10⁸ of Picia farinosa per gram, from about 2 x 10³ to 2 x 10⁸ of Dekkera bruxellensis per gram, from about 2 x 10³ to 4 x 10⁸ of Streptococci per gram, from about 8 x 10² to 6 x 10⁷ of Bacilli per gram, from about 6 x 10⁴ to 6 x 10⁷ of Staphylococci, from about 0.2% to 5% of lactic acid, from about 0.1% to 3% of acetic acid, from about 0.01% to 2% of succinic acid.

9. An animal feed additive according to any one of the preceding claims, **characterized in that** it has an energy content of about 2-15 MJ/kg on a dry weight basis.

10. An animal feed additive according to any one of the preceding claims, **characterized in that** said micro-organisms, enzymes, organic acids and bacteriocins have at least one, and preferably all, of the following properties:
1) Resistance to gastric acids,
2) Resistance to heat up to a maximum of 70°C,
3) Capability of being pelletized,
4) Stable in fresh and salt water,
5) Non-toxic for animals and humans,
6) Preventing infections,
7) Removing or curing infections,
8) Growth promoting,
9) Good colonization,
10) Bacteriocide effects, and
11) Immune enhancing effects.

11. An animal feed additive according to any one of the preceeding claims, **characterized in that** the animal feed additive is food stuffs for humans and that the animal is a human.

12. Use of an animal feed additive according to any one of the preceeding claims for preparing a preparation intended to maintain a normal bacterial flora and restore a pathological bacterial flora in an animal.

13. Use of an animal feed additive according to any one of claims 1 to 11 for preparing a preparation intended to increase the digestive uptake from the intestinal tract.

14. Use of an animal feed additive according to any one of claims 1 to 11 for preparing a preparation intended to improve the immune defense system in an animal.

15. Use of an animal feed additive according to any one of claims 1 to 11 for preparing a preparation intended to prevent weaning diarréa and other infections.

16. Use of an animal feed additive according to any one of claims 1 to 11 for preparing a preparation intended to reduce the pre-weaning mortality of piglets.

17. Use of an animal feed additive according to any one of claims 1 to 11for preparing a preparation intended to prevent diarréa and reduce the breeding time of slaughter pigs.

18. Use of an animal feed additive according to any one of claims 1 to 11 for preparing a preparation intended to prevent or reduce salmonella and/or campylobacter.

19. Use of an animal feed additive according to any one of claims 1 to 11 for preparing a preparation intended to prevent bacterial infections in schrimps and prawns.

20. Use of an animal feed additive according to claim 18 for preparing a preparation intended to prevent vibrio infections in schrimps.

21. Use of an animal feed additive as a human foodstuff according to any one of claims 1 to 11 for preparing a preparation intended to prevent acute bacterial infections in humans.

22. Use of an animal feed additive according to any one of claims 1 to 11 for preparing a preparation intended to reduce the amount of cholesterol and/or fat in an animal.

23. Use of an animal feed additive according to any one of claims 12 to 15, 18 and 22, wherein the animal feed additive is a human foodstuff and the animal is a human.

24. An animal feed comprising an animal feed additive according to any one of claims 1 to 11.

## Patentansprüche

1. Tierfutteradditiv für Tiere, **dadurch gekennzeichnet, dass** es umfasst mindestens einen der folgenden Mikroorganismen:
a) Pediococcus pentosaceus,
b) Pediococcus acidilactici.
und mindestens einen der folgenden Mikroorganismen:
c) Picia farinosa,
d) Dekkera bruxellensis,
und mindestens einen der folgenden Mikroorganismen:
e) Bacilli,
f) Streptococci,
g) Staphylococci,
und mindestens ebenso ein oder mehrere der folgenden hydrolytischen Enzyme:
h) proteolytische Enzyme,
i) Kohlenhydrat-aufspaltende Enzyme
j) lipolytische Enzyme,
k) Peroxidaseenzyme, und
l) Transferaseenzyme,
und mindestens eine der folgenden organischen Säuren:
m) Milchsäure,
n) Essigsäure,
o) Bernsteinsäure,
und mindestens zwei der folgenden Bakteriozine:
q) Pediocin A, Nisin, Pediocin AcH, P. acidilactice PAC 1.0

2. Tierfutteradditiv nach Anspruch 1, **dadurch gekennzeichnet, dass** es sämtliche der lebenden Mikroorganismen a)-g) umfasst.

3. Tierfutteradditiv nach mindestens einem der vorangehenden Ansprüche, **dadurch gekennzeichnet, dass** es sämtliche der hydrolytischen Enzyme h)-l) umfasst.

4. Tierfutteradditiv nach mindestens einem der vorangehenden Ansprüche. **dadurch gekennzeichnet, dass** es sämtliche der organischen Säuren m)-o) umfasst.

5. Tierfutteradditiv nach mindestens einem der vorangehenden Ansprüche, **dadurch gekennzeichnet, dass** es sämtliche der Bakteriozine Pediocin A, Nisin, Pediocin AcH und P. acidilactice PAC 1.0 umfasst.

6. Tierfutteradditiv nach mindestens einem der vorangehenden Ansprüche, **dadurch gekennzeichnet, dass** die proteolytischen Enzyme Enzyme mit Trypsin- und Peptidase-ähnlichen Aktivitäten beinhalten,
die Kohlenstoff-aufspaltenden Enzyme Amylase- und Cellulase-ähnliche Aktivitäten beinhalten,
die lipolytischen Enzyme Triacylglycerolase-ähnliche Aktivitäten beinhalten.
k) die Peroxidaseenzyme Catalase-ähnliche Aktivitäten beinhalten, und
l) die Transferaseenzyme Acyltransferase-ähnliche Aktivitäten beinhalten.

7. Tierfutteradditiv nach mindestens einem der vorangehenden Ansprüche, **dadurch gekennzeichnet, dass** es ebenso ein Tierfutter umfasst, das per se bekannt sein kann.

8. Tierfutteradditiv nach mindestens einem der vorangehenden Ansprüche, **dadurch gekennzeichnet, dass** es umfasst etwa 8 bis 15% Wasser, etwa 15 bis 30% Gesamtprotein, etwa 0,5 bis 5% Gesamtlipide, etwa 5 bis 20% Fasern, etwa 8 bis 20% Aschen (Mineralien), etwa 30 bis 50% lösliche Nicht-Stickstoff-Bestandteile, etwa 6 x 10⁴ bis 3 x 10⁹ lebende Pediococcus pentosaceus pro Gramm, 1 x 10³ bis 1 x 10⁷ lebende Pediococcus acidilactici pro Gramm, etwa 2 x 10³ bis 10⁸ Picia farinosa pro Gramm, etwa 2 x 10³ bis 2 x 10⁸ Dekkera bruxellensis pro Gramm, etwa 2 x 10³ bis 4 x 10⁸ Streptococci pro Gramm, etwa 8 x 10² bis 6 x 10⁷ Bacilli pro Gramm, etwa 6 x 10⁴ bis 6 x 10⁷ Staphylococci, etwa 0,2% bis 5% Milchsäure, etwa 0,1% bis 3% Essigsäure, etwa 0,01% bis 2% Bernsteinsäure.

9. Tierfutteradditiv nach mindestens einem der vorangehenden Ansprüche, **dadurch gekennzeichnet, dass** es einen Energiegehalt von etwa 2-15 MJ/kg auf Trockengewichtsbasis aufweist.

10. Tierfutteradditiv nach mindestens einem der vorangehenden Ansprüche, **dadurch gekennzeichnet, dass** die Mikroorganismen, Enzyme, organischen Säuren und Bakteriozine mindestens eine und vorzugsweise sämtliche der folgenden Eigenschaften aufweisen:
1) Beständigkeit gegenüber Magensäuren,
2) Beständigkeit gegenüber Wärme bis zu einem Maximum von 70°C,
3) Fähigkeit, pelletisiert zu werden,
4) stabil in frischem und Salzwasser,
5) nicht toxisch gegenüber Tieren und Menschen,
6) Verhinderung von Infektionen,
7) Beseitigung oder Heilung von Infektionen,
8) wachstumsfördernd,
9) gute Ausbreitung,
10) bakteriozide Effekte, und
11) immunverstärkende Effekte.

11. Tierfutteradditiv nach mindestens einem der vorangehenden Ansprüche, **dadurch gekennzeichnet, dass** das Tierfutteradditiv Nahrungsmittel für Menschen ist, und dass das Tier ein Mensch ist.

12. Verwendung eines Tierfutteradditivs nach mindestens einem der vorangehenden Ansprüche zur Herstellung eines Präparats, welches dazu dient, bei einem Tier eine normale Bakterienflora beizubehalten und eine pathologische Bakterienflora wiederherzustellen.

13. Verwendung eines Tierfutteradditivs nach mindestens einem der Ansprüche 1 bis 11 zur Herstellung eines Präparats, welches dazu dient, die Verdauungsaufnahme aus dem Intestinaltrakt zu erhöhen.

14. Verwendung eines Tierfutteradditivs nach mindestens einem der Ansprüche 1 bis 11 zur Herstellung eines Präparats, das dazu dient, das Immunabwehrsystem bei einem Tier zu verbessern.

15. Verwendung eines Tierfutteradditivs nach mindestens einem der Ansprüche 1 bis 11 zur Herstellung eines Präparats, das dazu dient, beim Entwöhnen auftretende Diarrhöe und andere Infektionen zu verhindern.

16. Verwendung eines Tierfutteradditivs nach mindestens einem der Ansprüche 1 bis 11 zur Herstellung eines Präparats, das dazu dient, die Pre-Entwöhnungssterblichkeit von Ferkeln zu verringern.

17. Verwendung eines Tierfutteradditivs nach mindestens einem der Ansprüche 1 bis 11 zur Herstellung eines Präparats, das dazu dient, Diarrhöe zu verhindern und die Aufzuchtzeit von Schlachtschweinen zu verringern.

18. Verwendung eines Tierfutteradditivs nach mindestens einem der Ansprüche 1 bis 11 zur Herstellung eines Präparats, das dazu dient, Salmonellen und/oder Campylobakterien zu verhindern oder zu verringern.

19. Verwendung eines Tierfutteradditivs nach mindestens einem der Ansprüche 1 bis 11 zur Herstellung eines Präparats, das dazu dient, bakterielle Infektionen in Schrimps und Garnelen zu verhindern.

20. Verwendung eines Tierfutteradditivs nach Anspruch 18, zur Herstellung eines Präparats, das dazu dient, Vibrioinfektionen in Schrimps zu verhindern.

21. Verwendung eines Tierfutteradditivs als Humannahrungsmittel nach mindestens einem der Ansprüche 1 bis 11 zur Herstellung eines Präparats, das dazu dient, akute bakterielle Infektionen bei Menschen zu verhindern.

22. Verwendung eines Tierfutteradditivs nach mindestens einem der Ansprüche 1 bis 11 zur Herstellung eines Präparats, das dazu dient, die Menge an Cholesterol und/oder Fett bei einem Tier zu verringern.

23. Verwendung eines Tierfutteradditivs nach mindestens einem der Ansprüche 12 bis 15, 18 und 22, wobei das Tierfutteradditiv ein Humannahrungsmittel ist und das Tier ein Mensch ist.

24. Tierfutter, umfassend ein Tierfutteradditiv nach mindestens einem der Ansprüche 1 bis 11.

## Revendications

1. Additif pour aliment pour animaux, **caractérisé en ce qu'**il comprend au moins l'un des microorganismes suivants :
a) Pediococcus pentosaceus,
b) Pediococcus acidilactici,
et au moins l'un des microorganismes suivants :
c) Picia farinosa,
d) Dekkera bruxellensis,
et au moins l'un des microorganismes suivants :
e) Bacilli,
f) Streptococci;
g) Staphylococci,
et au moins aussi l'une ou plusieurs des enzymes hydrolytiques suivantes :
h) enzymes protéolytiques,
i) enzymes clivant les glucides,
j) enzymes lipolytiques,
k) enzymes peroxydases, et
l) enzymes transférases,
et au moins l'un des acides organiques suivants :
m) acide lactique,
n) acide acétique,
o) acide succinique,
et au moins deux des bactériocines suivantes :
q) pédiocine A, nisine, pédiocine AcH, P. acidilactice PAC 1.0.

2. Additif pour aliment pour animaux suivant la revendication 1, **caractérisé en ce qu'**il comprend tous les microorganismes vivants a) à g).

3. Additif pour aliment pour animaux suivant l'une quelconque des revendications précédentes, **caractérisé en ce qu'**il comprend toutes les enzymes hydrolytiques h) à l).

4. Additif pour aliment pour animaux suivant l'une quelconque des revendications précédentes, **caractérisé en ce qu'**il comprend tous les acides organiques m) à o).

5. Additif pour aliment pour animaux suivant l'une quelconque des revendications précédentes, **caractérisé en ce qu'**il comprend toutes les bactériocines pédiocine A, nisine, pédiocine AcH et P. acidilactice PAC 1.0.

6. Additif pour aliment pour animaux suivant l'une quelconque des revendications précédentes, **caractérisé en ce que** lesdites enzymes protéolytiques comprennent des enzymes ayant des activités de type trypsine et de type peptidase,
lesdites enzymes clivant les glucides comprennent des activités de type amylase et de type ceiluiase,
lesdites enzymes lipolytiques comprennent des activités de type triacylglycérolase,
k) lesdites enzymes peroxydases comprennent des activités de type catalase, et
l) lesdites enzymes transférases comprennent des activités de type acyltransférase.

7. Additif pour aliment pour animaux suivant l'une quelconque des revendications précédentes, **caractérisé en ce qu'**il comprend aussi un aliment pour animaux qui peut être connu en soi.

8. Additif pour aliment pour animaux suivant l'une quelconque des revendications précédentes, **caractérisé en ce qu'**il comprend environ 8 à 15% d'eau, environ 15 à 30% de protéine totale, environ 0,5 à 5% de lipides totaux, environ 5 à 20% de fibres, environ 8 à 20% de cendres (minéraux), environ 30 à 50% de matière soluble non azotée, environ 6x10⁴ à 3x10⁹ de Pediococcus pentosaceus vivant par gramme, d'environ 1x10³ à 1x10⁷ Pediococcus acidilactici vivant par gramme, d'environ 2x10³ à 10⁸ de Picia farinosa par gramme, d'environ 2x10³ à 2x10⁸ de Dekkera bruxellensis par gramme, d'environ 2x10³ à 4x10⁸ de Streptococci par gramme, d'environ 8x10² à 6x10⁷ de Bacilli par gramme, d'environ 6x10⁴ à 6x10⁷ de Staphylococci, d'environ 0,2% à 5% d'acide lactique, d'environ 0,1% à 3% d'acide acétique, d'environ 0,01 à 2% d'acide succinique.

9. Additif pour aliment pour animaux suivant l'une quelconque des revendications précédentes, **caractérisé en ce qu'**il a une teneur énergétique d'environ 2 à 15 MJ/kg sur une base de poids sec.

10. Additif pour aliment pour animaux suivant l'une quelconque des revendications précédentes, **caractérisé en ce que** lesdits microorganismes, enzymes, acides organiques et bactériocines ont au moins l'une de propriétés, et de préférence toutes les propriétés suivantes :
1) résistance aux acides gastriques,
2) résistance à la chaleur jusqu'à un maximum de 70°C,
3) aptitude à être mis sous forme de granulés,
4) stabilité dans l'eau douce ou salée,
5) absence de toxicité pour les animaux et les humains,
6) prévention d'infections,
7) élimination ou guérison d'infections,
8) activation de la croissance,
9) bonne colonisation,
10) effets bactéricides, et
11) effets immunostimulants.

11. Additif pour aliment pour animaux suivant l'une quelconque des revendications précédentes, caractérisé en ce l'additif pour aliment pour animaux est une denrée alimentaire pour les humains et que l'animal est un humain.

12. Utilisation d'un additif pour aliment pour animaux suivant l'une quelconque des revendications précédentes, pour la préparation d'une composition destinée à maintenir une flore bactérienne normale et à restaurer une flore bactérienne pathologique chez un animal.

13. Utilisation d'un additif pour aliment pour animaux suivant l'une quelconque des revendications 1 à 11, pour la préparation d'une composition destinée à augmenter l'absorption par digestion des voies intestinales.

14. Utilisation d'un additif pour aliment pour animaux suivant l'une quelconque des revendications 1 à 11, pour la préparation d'une composition destinée à améliorer le système de défense immun chez un animal.

15. Utilisation d'un additif pour aliment pour animaux suivant l'une quelconque des revendications 1 à 11, pour la préparation d'une composition destinée à prévenir des diarrhées de sevrage et d'autres infections.

16. Utilisation d'un additif pour aliment pour animaux suivant l'une quelconque des revendications 1 à 11, pour la préparation d'une composition destinée à réduire la mortalité avant le sevrage chez les porcelets.

17. Utilisation d'un additif pour aliment pour animaux suivant l'une quelconque des revendications 1 à 11, pour la préparation d'une composition destinée à prévenir la diarrhée et à réduire le temps d'élevage des porcs pour abattage.

18. Utilisation d'un additif pour aliment pour animaux suivant l'une quelconque des revendications 1 à 11, pour la préparation d'une composition destinée à prévenir ou à réduire les salmonelles et/ou les campylobacters.

19. Utilisation d'un additif pour aliment pour animaux suivant l'une quelconque des revendications 1 à 11, pour la préparation d'une composition destinée à prévenir des infections bactériennes chez les crevettes et les bouquets

20. Utilisation d'un additif pour aliment pour animaux suivant la revendication 18, pour la préparation d'une composition destinée à prévenir des infections par des vibrions chez les crevettes.

21. Utilisation d'un additif pour aliment pour animaux en tant que produits alimentaires pour les humains suivant l'une quelconque des revendications 1 à 11, pour la préparation d'une composition destinée à prévenir des infections bactériennes aiguës chez les humains.

22. Utilisation d'un additif pour aliment pour animaux suivant l'une quelconque des revendications 1 à 11, pour la préparation d'une composition destinée à réduire la quantité de cholestérol et/ou de graisses chez un animal.

23. Utilisation d'un additif pour aliment pour animaux suivant l'une quelconque des revendications 12 à 15, 18 et 22, dans laquelle l'additif pour aliment pour animaux est un produit alimentaire pour les humains et que l'animal est un être humain.

24. Aliment pour animaux comprenant un additif pour aliment pour animaux suivant l'une quelconque des revendications 1 à 11.
